# EUROPÄISCHE PATENTSCHRIFT

(11) **EP 0 672 834 B1**
(45) Veröffentlichungstag und Bekanntmachung des Hinweises auf die Patenterteilung: **03.05.2000**
(21) Anmeldenummer: 95101736.7
(22) Anmeldetag: 09.02.1995
(51) Int. Cl.: F15C 4/00, F04B 43/04

(54) **Mikro-Fluidmanipulator**
Micro fluid manipulator
Micro-manipulateur pour fluides

(30) Priorität: 17.02.1994 DE 4405026
(43) Veröffentlichungstag der Anmeldung: 20.09.1995
(73) Patentinhaber: Forschungszentrum Rossendorf e.V., D-01474 Rossendorf (DE)
(72) Erfinder: Pham, Minh Tan, Dr., D-01324 Dresden (DE); Howitz, Steffen, Dr., D-01309 Dresden (DE)

(56) Entgegenhaltungen:
- EP-A- 0 025 005
- DE-A- 4 140 533
- PATENT ABSTRACTS OF JAPAN vol. 6, no. 29 (M-113) 20. Februar 1982 & JP-A-56 146 765 (HITACHI) 14. November 1981
- MICRO ELECTRO MECHANICAL SYSTEMS, Februar 1991, NARA, JAPAN Seiten 271 - 276 RICHTER 'electrohydrodynamic pumping and flow measurement.'
- ANALYTICAL CHEMISTRY, Bd.65, Nr.19, 1. Oktober 1993 Seiten 2637 - 2642 EFFENHAUSER 'glass chips for high-speed capillary electrophoresis separation with submicrometer plate heights.'
- TRANSDUCERS '93, Juni 1993, YOKOHAMA, JAPAN Seiten 403 - 406 HARRISON 'miniaturized chemical analysis systems based on elctrophoretic separations and elctroosmotic pumping'

## Beschreibung

Die Erfindung betrifft einen Mikro-Fluidmanipulator zum steuerbaren Transportieren, Dosieren, und Mischen von Fluiden im Submikroliter-Bereich ohne mikromechanische Membranpumpen und Ventile. Sie betrifft im besonderen einen Mikro-Fluidmanipulator zur steuerbaren drucklosen Dosierung vorzugsweise hochkonzentrierter Medikamente.

Im Bereich der Biomedizintechnik sind bezüglich des Fluidhandlings implantierbare Medikamentenpumpen mit Ein- und Auslaß-Restriktion in "Open loop-Geräten", steuerbare implantierbare Medikamentendosiersysteme auf der Basis der Treibgaspumpe mit Ventil und die Kolbenpumpe ohne Ventil bekannt. Die Realisierung solcher Systeme basiert bisher auf feinmechanischen Fertigungstechnologien. Probleme ergeben sich hierbei aus Fördermechanismen und Leckraten. Hinzu kommt die Einschränkung der kommerziellen Verfügbarkeit durch hohe Kosten. Zum Mikrofluidhandling in chemischen Mikroanalysesystemen und -verfahren, die gegenwärtig einen aktuellen Gegenstand der Forschung und Entwicklung darstellen, kommen bisher ausschließlich mikromechanische Membranpumpen und Ventile auf der Basis der Si-Technologie zum Einsatz [Van der Schoot et al., A silicon integrated miniature chemical analysis System, Sensors and Actuators B6 (1992) 57-60); M. Esashi, Integrated micro flow control Systems, Proc. Fluid Toyota Conference, Nissin 1989, pp. 21/1-21/16]. Momentan erkennbar ist zum einen das Problem daß mikromechanische Ventile nicht absolut dicht schließen können. Leckraten von 0,25 bis 2,5 µl/min sind in der zitierten Arbeit angegeben worden. Die Nachweisgrenze ist dadurch eingeschränkt. Ein zweites Problem ist die aufwendige Herstellungs- und Montagetechnologie, da die Pumpen- und Ventilstrukturen sehr kompliziert sind.
Bezüglich des Mikro-Fluidhandlings sind weiterhin Mikropumpen zum Transportieren von Fluiden nach dem Prinzip der Elektrohydrodynamik [A. Richter, A. Plettner, K. A. Hofmann and H. Sandmaier, Electrohydrodynamic pumping and flow measurement, 4th IEEE Workshop on Micro Electro-Mechanical Systems, 30.1 - 2.2.1991, Nara, Japan] und des elektroosmotischen und elektrophoretischen Pumpens [C. S. Effenhauser, A. Manz und H. M. Widmer, Glass chips for high-speed capillary electrophoresis separations with submicrometer plate heights, Anal. Chem. 65 (1993) 2637-2642; D. J. Harrison, Z. Fan, K. Seiler and K. Flurri, Miniaturized Chemical Analysis Systems based on Electrophoretic Separations and Electroosmotic Pumping, 7th Inter. Conf. on Solid-State Sensors and Actuators, June 7 - 10, 1993 Yokohama, Japan, Digest of technical papers, pp. 403-406] vorgestellt worden. In den zitierten Arbeiten werden Labormuster auf der Basis der mikrotechnischen Verfahren der Si-Technologie beschrieben. Beiden Prinzipien gemeinsam ist die Voraussetzung, daß ein starkes elektrisches Feldes innerhalb des Fluids besteht, für dessen Erzeugung Spannungen von einigen hundert Volt bis Kilovolt und Elektroden in direktem Kontakt mit dem Fluidmedium erforderlich sind. Der Einsatz beschränkt sich auf nichtwässrige oder niederleitfähige Fluidmedien. Darüber hinaus besteht das Problem der Leckrate, die die Dosiergenauigkeit im Submikroliter-Bereich einschränkt.

Der Erfindung liegt die Aufgabe zugrunde, einen Mikro-Fluid-Manipulator zum Steuerbaren Transportieren, Dosieren, Injizieren und Mischen von Fluiden, insbesondere für Anwendungen im Bereich der Biomedizintechnik und der chemischen Mikrosensorik unter der Prämisse zu schaffen, daß die oben angeführten Probleme mikromechanischer Pumpen und Ventile ausgeschlossen sind, und damit ein besserer und sichererer Funktionsbetrieb, eine erhöhte Genauigkeit bezüglich des Dosiervolumens sowie eine verbesserte Unterscheidbarkeit zwischen Ein- und Aus-Zustand des Gesamtsystems erzielt werden.

Die Aufgabe wird durch die in den Patentansprüchen dargestellten Ausführungsformen der Erfindung mit mikrotechnisch hergestellten Komponenten und deren Montage zu einem kompakten Modul mittels mikrosystemtechnischer Aufbau- und Verbindungstechniken gelöst.

Mit der Erfindung wird das Modul zum Erzeugen des Fluidstrahls, hier als Mikrotropfen-Emitter benannt, in eine planare Konstruktion gebracht, welche Voraussetzung für eine Verkopplung mit weiteren modularen mikrotechnisch hergestellten Komponenten auf der Basis der Si-Glas-Technologie darstellt. Eine mit einem Dosierfluid-Vorrat verbundene Mikrokapillare ist als Mikrotropfenemitter ausgebildet, indem sie vor ihrer als Emitterdüse dienenden Austrittsöffnung zu eine Fluidkammer erweitert und deren Wandung mit einer piezoelektrischen Energiequelle gekoppelt ist. Die kurze Entfernung der Fluidkammer zur Emitterdüse ermöglicht, daß bei einer Spannungbeaufschlagung des Piezo-Aktors das Fluid als Tröpfchen aus der Emitterdüse heraus gestoßen wird. Mit diesem Mikrotropfen-Emitter lassen sich über die Dimensionierung der Emitterdüse und die Frequenz des Piezo-Aktors Fluidtröpfchen in Volumen und Anzahl exakt steuern. Das Dosierfluid kann dazu u.a. aus einem Vorratsgefäß oder auch im Zuge einer durchlaufenden Kapillare dem Mikrotropfenemitter zugeführt, bzw. durch diesen hindurchgeführt werden. Eine fluidische Diode, die in Flugweite des emittierten Fluidstrahls so plaziert ist, daß der vom Mikrotropfen-Emitter emittierte Fluidstrahl auf ihren Eingang trifft, wird zur Einkopplung des freitragenden Fluid-Tröpfchenstrahls in ein weiteres fließendes Fluidmedium eingesetzt. Diese Mikrofluiddiode besteht aus einer beidseitig offenen Mikrokapillare oder einem System von dicht nebeneinander angeordneten Mikrokapillaren, welches im Grenzfall ein zweidimensionales Siebnetz darstellen kann. Die im Strömungskanal strömende Flüssigkeit benetzt beim Passieren der Mikrofluiddiode zwangsläufig die anliegenden Enden der Mikrokapillaren und spreitet bis zu deren gegenüberliegenden Enden empor. Unter dem Einfluß der Oberflächenspannung der emporstreitenden Flüssigkeit bildet sich auf jeder dieser Mikrokapillaren auf der Seite der Tröpfchenkammer eine definierte Flüssigkeit-Gas-Grenzfläche, ein sogenannter Meniskus aus. Mit der Ausbildung jedes Meniskus wird der Vorgang des Spreitens der Flüssigkeit in der entsprechenden Mikrokapillare abrupt abgeschlossen und das Herausfließen der strömenden Flüssigkeit aus dieser Mikrokapillare vollständig verhindert. Wird nun eine zweite Flüssigkeit auf den Meniskus aufgesetzt, kommt es zur schlagartigen Benetzung zwischen beiden Flüssigkeiten und die zweite Flüssigkeit kann ohne Hinderung durch die Mikrokapillare in das Innere des Strömungskanales gelangen. Der ungehinderte Eintritt der zweiten Flüssigkeit über den Meniskus der ersten Flüssigkeit in den Strömungskanal erfolgt dabei über Diffusion und Konvektion.
Durch eine entsprechende Behandlung ihrer ein- und ausgangsseitigen Kapillaroberflächen kann die Dimensionierung der Kapillarweite der Mikrofluiddiode in Abhängigkeit von Dosier- und Zielfluid weiter variiert werden, so daß sie als semipermeable Membran ein Eindringen des äußeren Zielfluids in den Mikro - Fluidmanipulator mit Sicherheit verhindert, den Transport des Dosierfluids in das Zielfluid jedoch garantiert.

Unter Bezugnahme auf die zugehörigen Zeichnungen wird die Erfindung an folgenden Ausführungsbeispielen näher erläutert

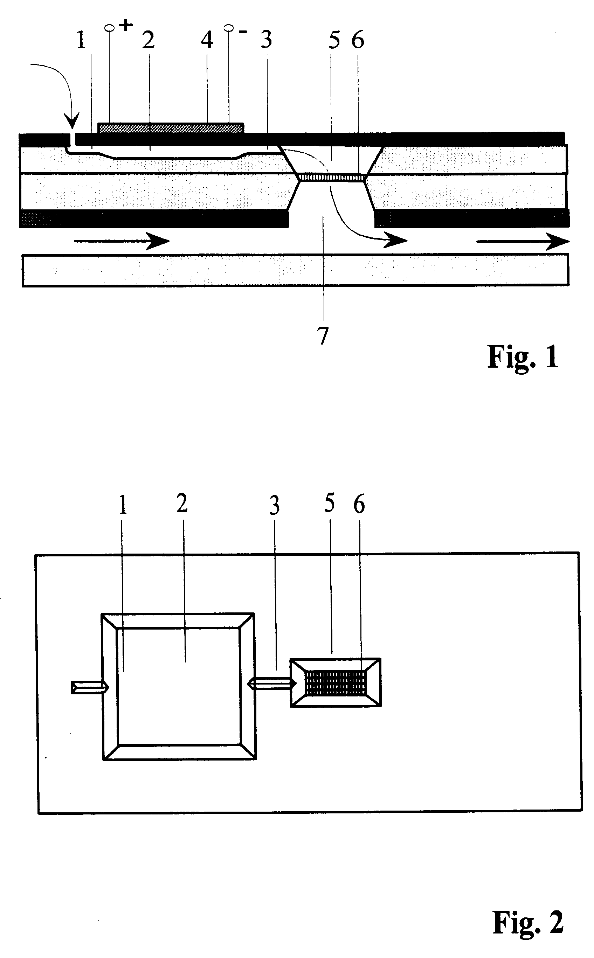

Fig. 1 zeigt die Schnittdarstellung der planaren Konstruktion des erfindungsgemäßen Mikro -Fluidmanipulators, welcher als ein zusammengesetztes Modul mit zwei Funktionseinheiten, dem Mikro-Tropfenemitter und der Mikrofluiddiode realisiert ist. Die mit einer externen oder aber in den Chip integrierten Vorratskammer für das Dosierfluid verbundene Kapillare 1, die Fluidkammer 2, die Emitterdüse 3 und der piezoelektrisch angetriebene, auf der Fluidkammer 2 plazierte Aktor 4, bilden den Mikrotropfen-Emitter. Durch eine Spannungsbeaufschlagung des Aktors 4 werden Dosierfluidtröpfchen in die, aus einem gasgefüllten, geschlossenen Hohlraum bestehende Tröpfchenkammer 5 emittiert und benetzen dort die Eingangsoberfläche der Mikrofluiddiode 6. Diese Eingangsoberfläche kann z.B. zur Unterstützung der Dosierung einer wässrigen Lösung in ein wässriges Zielfluid hydrophil ausgestattet sein, während die Ausgangsseite hydrophob beschichtet ist. Auch allein durch die Dimensionierung der Kapillaren und dem sich ausbildenden Meniskus ist ein ungehinderter Übergang des Dosierfluids in das äußere Zielfluid gewährleistet, wohingegen das äußere, nahezu drucklose Fluid die Mikrofluiddiode nicht passieren kann. Die Mikrofluiddiode 5 ist hier beispielhaft als eine in Si durchgeätzte Netzstraktur mit einer Maschenweite von z.B. 30 x 30 µm² ausgebildet. Die ausgangsseitige Öffnung 7 der Mikrofluiddiode stellt die Verbindung zu einem fließenden Zielfluid her.

Fig. 2 zeigt eine schematische Darstellung des erfindungsgemäßen Mikro-Fluidmanipulator mit der beispielhaften Dimensionierung: Chipgröße von 18 x 12 mm², Fluidkammer von 1000 x 1000 x 200 µm³, Kapillarquerschnitt von 200 x 200 µm², Öffnung der Emitterdüse von 92 x 92 µm², Maschenweite des Diodennetzes von 30 x 30 µm².

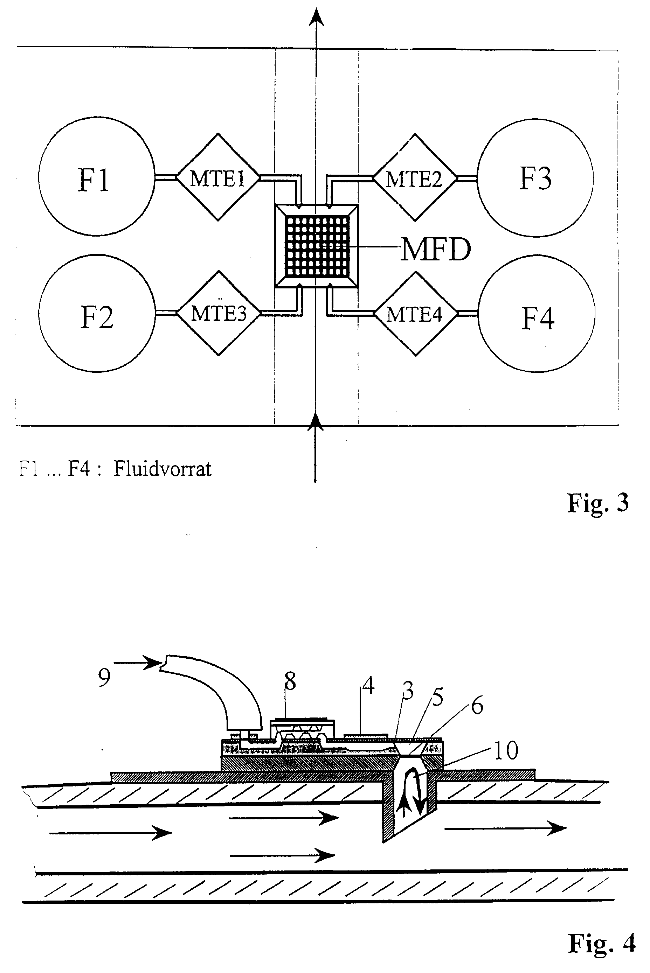

Fig. 3 zeigt die schematische Darstellung für ein zweites Ausführungsbeispiel des erfindungsgemäßen Mikro-Fluidmanipulators. Angegeben ist die beispielhafte Konstruktion eines 4-Kanal-Fluidinjektors. Dieser Fluidinjektor besteht aus mehreren, mit separaten Fluidvortatskammern F1...4 verbundenen Mikrotropfen-Emittern (MTE1...4), die alle in die, den Eingang der Mikrofluiddiode bildende Tröpfchenkammer 2 der gemeinsamen Mikrofluiddiode 1 münden. Die Funktion dieses Fluidinjektors ähnelt einem mechanischen Multikanal-Ventil, das an mehrere mechanische Membranpumpen angeschlossen ist. Der wesentliche Unterschied besteht darin, daß der Fluidinjektor mit der erfindungsgemäßen Lösung leckfrei arbeitet und somit eine bessere Dosiergenauigkeit gestattet. Aus dem angeführten Beispiel ist ein Multikanal-Fluidinjektor mit beliebiger Anzahl an Einzelkomponenten selbstverständlich ableitbar.

Eine auf der Basis des erfindungsgemäßen Mikro-Fluidmanipulator aufgebaute Medikamenten-Mikrodosiervorrichtung die besonders effektiv und kostengünstig herstellbar ist, und eine erhöhte Dosiergenauigkeit bei einer Verkleinerung der Dosiermenge aufweist sowie einen sichereren Furktionsbetrieb ermöglicht, ist in Fig. 4 beispielhaft dargestellt. Die Konstruktion besteht insgesamt aus mikrotechnisch hergestellten Komponenten, deren Montage zu einem kompakten Modul mittels mikrosystemtechnischer Aufbau- und Verbindungstechniken erfolgt. Der von dem piezoelektrischen Aktor 4 angetriebene Mikrotropfen-Emitter ist über ein zum Ausgleich eines eventuellen Überdruckes vorgesehenes Sicherheitsventil 8 an ein Vorratsgefäß für das Medikamentenfluid 9 angeschlossen. Er mündet mit seiner Emitterdüse 3 in die Tröpfcherkammer 5, welche im Zustand "Dosieren-Aus" fluidleer ist. Der Boden der Tröpfchenkammer 5 stellt den Eingang der Mikro-Fluiddiode 6 dar, welche über die Kanüle 10 das Dosiergut in das fließende biologische Fluidmedium führt.

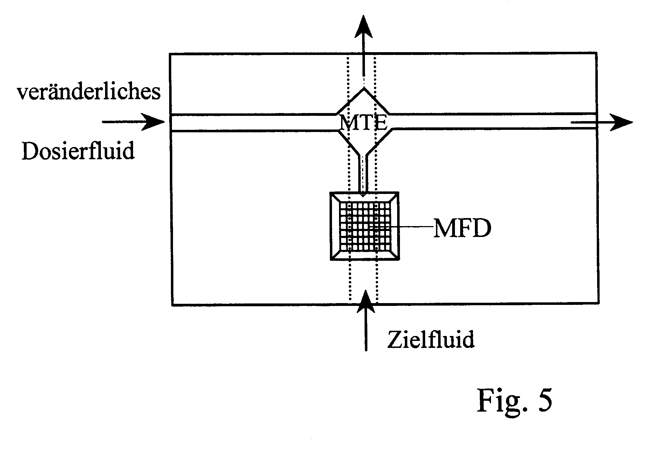

In Fig. 5 ist schematisch ein Beispiel aufgezeigt, bei dem der Mikrofluid-Manipulator im Sinne einer Probenahme aus einem fließenden, veränderlichen Dosierfluid eingesetzt wird. Dazu wird der Mikrotropfen-Emitter in den Verlauf der Dosierfluid-Strömungskapillare so integriert, daß er ständig und unabhängig von seiner eigenen Ansteuerung vom veränderlichen Dosierfluid durchströmt wird. Erst bei Meßbedarf wird der Mikrotropfenemitter MTE aktiviert und emittiert das zu diesem Zeitpunkt anliegende aktuelle Dosierfluid über die Mikro-Fluiddiode in den Zielfluid-Strömungskanal.

Mit dem erfindungsgemäßen Mikro - Fluidmanipulator gelingt es, ein neues Konzept zum steuerbaren Transportieren, Dosieren, Injizieren und Mischen von Fluiden ohne mikromechanische Membranpumpen und Ventile zu realisieren. Der besondere Vorteil der sich aus der erfindungsgemäßen Lösung ergibt, besteht in der vollständigen fluidischen Entkopplung zwischen dem Mikrotropfen-Emitter und der Mikrofluiddiode über die Tröpfchenkammer. Daraus resultiert eine absolute Unterscheidbarkeit zwischen dem Ein-Zustand und dem Aus-Zustand, so daß im Aus-Zustand eine totale Leckfreiheit erzielbar ist. Dies ermöglicht über die Steuerfrequenz des piezoelektrischen Aktors und die Dimensionierung der Emitterdüse eine Dosiergenauigkeit im Pikoliter-Bereich. Der erfindungsgemäße Mikro -Fluidmanipulator zeichnet sich durch eine einfache Konstruktion aus, woraus sich Vorteile bezüglich Miniaturisierung und Herstellung ergeben. Mit dem hier eingeführten Prinzip der Einkopplung eines freitragenden Fluid-Tröpfchenstrahls in ein fließendes geschlossenes Fluidmedium wird ein neues Anwendungsfeld in der Biomedizintechnik und der chemischen Mikrosensorik ermöglicht.
Darüber hinaus sind selbstverständlich Anwendungen auf allen technischen Gebieten möglich, bei denen das Problem einer miniaturisierten Fluiddosierung besteht, wie z.B. in der Pharma- und Feinchemikalien-Industrie, der Labor- und Weltraumtechnik, der Bio-und Gentechnologie sowie in der Automobilindustrie z.B. für Kat-Systeme.

## Patentansprüche

1. Mikro-Fluidmanipulator zum steuerbaren Transportieren, Dosieren, Injizieren und Mischen von Fluiden im Pikoliter- bis Mikroliterbereich, bestehend aus einer planaren Anordung eines mit einem Dosierfluid-Vorrat verbundenen Mikrotropfenemitters und einer, über eine, aus einem geschlossenen, gasgefüllten Hohlraum bestehende Tröpfchenkammer (5) mit dem Mikrotropfenemitter verbundenen, nur in einer Richtung fluiddurchlässigen Mikrofluiddiode (6), wobei Mikrotropfenemitter und Mikrofluiddiode (6) so angeordnet sind, daß der vom Mikrotropfenemitter emittierte Fluidtropfen auf den Eingang der Mikrofluiddiode (6) trifft, und daß die Mikrofluiddiode (6) ausgangsseitig in ein strömendes Zielfluid mündet.

2. Mikro-Fluidmanipulator nach Anspruch 1, dadurch gekennzeichnet, daß der Mikrotropfenemitter aus einer Mikrokapillare (1) besteht, die vor ihrer als Emitterdüse (3) fungierenden Austrittsöffnung zu einer Fluidkammer (2) erweitert ist, deren Wandung an eine piezoelektrisch steuerbare Energiequelle angekoppelt ist.

3. Mikro-Fluidmanipulator nach Anspruch 1, dadurch gekennzeichnet, daß der Mikrotropfenemitter in einer von einem veränderlichen Dosierfluid durchströmten Kapillare (1) angeordnet ist.

4. Mikro-Fluidmanipulator nach Anspruch 1, dadurch gekennzeichnet, daß die Mikrofluiddiode (6) aus einer beidseitig offenen Mikrokapillare (1) oder einem System von dicht nebeneinander angeordneten beidseitig offenen Mikrokapillaren (1) besteht, wobei die Kapillar-Oberflächen eingangsseitig hydrophil und ausgangsseitig hydrophob ausgestattet sind.

5. Mikro-Fluidmanipulator nach Anspruch 1 dadurch gekennzeichnet, daß dessen Komponenten aus Silizium, Glas, Keramik, Metall oder aus einer Kombination von diesen Materialien aufgebaut und durch mikrotechnische Verfahren und mikrosystemtechnische Aufbau- und Verbindungstechniken hergestellt sind.

6. Mikro-Fluidmanipulator nach Anspruch 1 dadurch gekennzeichnet, daß die Oberfläche der Tröfchenkammer (5) hydrophob ist.

7. Mikro-Fluidmanipulator nach Anspruch 1 dadurch gekennzeichnet, daß mehrere Mikrotropfenemitter und Mikrofluiddioden (6) zu einer mehrschichtigen Kaskade kombiniert sind.

8. Mikro-Fluidmanipulator nach Anspruch 1, insbesondere zur steuerbaren, drucklosen Dosierung vorzugsweise hochkonzentrierter Medikamente für den Einsatz in der Biomedizin, dadurch gekennzeichnet, daß zwischen dem Vorratsgefäß und dem Mikrotropfenemitter ein druckkompensiertes Sicherheitsventil (8) angeordnet ist, und daß der Ausgang der Mikrofluiddiode (6) in eine biokompatible Kopplungskanüle (10) mündet.

## Claims

1. Micro fluid manipulator for controllable transporting, metering, injecting and mixing of fluids in the picolitre to microlitre range, consisting of a planar arrangement of a microdroplet emitter, which is connected to a metering fluid reservoir, and of a micro fluid diode (6) which is connected to the microdroplet emitter via a droplet chamber (5), consisting of a closed, gas-filled cavity, and which allows fluid to pass through only in one direction, the microdroplet emitter and micro fluid diode (6) being arranged in such a way that the fluid droplet emitted by the microdroplet emitter strikes the inlet of the micro fluid diode (6) and in such a way that the outlet of the micro fluid diode (6) opens into a stream of target fluid.

2. Micro fluid manipulator according to Claim 1, characterized in that the microdroplet emitter consists of a microcapillary (1) which, before its outlet opening functioning as emitter nozzle (3), is widened to form a fluid chamber (2) whose wall is coupled to a piezoelectrically controllable energy source.

3. Micro fluid manipulator according to Claim 1, characterized in that the microdroplet emitter is arranged in a capillary (1) through which there flows a variable metering fluid.

4. Micro fluid manipulator according to Claim 1, characterized in that the micro fluid diode (6) consists of a microcapillary (1) open at both ends or of a system of microcapillaries (1) arranged next to one another and open at both ends, the capillary surfaces being made hydrophilic on the inlet side and hydrophobic on the outlet side.

5. Micro fluid manipulator according to Claim 1, characterized in that its components are made of silicon, glass, ceramic, metal or of a combination of these materials and are produced by microtechnology procedures and microsystem-based construction and assembly techniques.

6. Micro fluid manipulator according to Claim 1, characterized in that the surface of the droplet chamber (5) is hydrophobic.

7. Micro fluid manipulator according to Claim 1, characterized in that a plurality of microdroplet emitters and micro fluid diodes (6) are combined to a form a multilayer cascade.

8. Micro fluid manipulator according to Claim 1, in particular for controllable, pressureless metering of preferably highly concentrated medicaments for use in biomedicine, characterized in that a pressure-compensated safety valve (8) is arranged between the storage reservoir and the microdroplet emitter, and in that the outlet of the micro fluid diode (6) opens into a biocompatible coupling cannula (10).

## Revendications

1. Micro-manipulateur pour fluides pour le transport, le dosage, l'injection et le mélange contrôlables de fluides dans le domaine du picolitre au microlitre, se composant d'un dispositif plan d'un émetteur de microgouttes connecté à une réserve de fluide de dosage et d'une diode microfluide (6) perméable aux fluides uniquement dans une direction, reliée à l'émetteur de microgouttes par l'intermédiaire d'une chambre à gouttelettes (5) se composant d'un espace creux fermé rempli de gaz, l'émetteur de microgouttes et la diode microfluide (6) étant disposés de telle sorte que la goutte de fluide émises par l'émetteur de microgouttes arrive à l'entrée de la diode microfluide (6), et que la diode microfluide (6) débouche du côté de la sortie dans un fluide cible en mouvement.

2. Micro-manipulateur pour fluides selon la revendication 1, caractérisé en ce que l'émetteur de microgouttes se compose d'un microcapillaire (1), qui est élargi avant son ouverture de sortie se prolongeant en buse d'émission (3) dans une chambre de fluide (2), dont la paroi est couplée à une source d'énergie contrôlable par voie piezoélectrique.

3. Micro-manipulateur pour fluides selon la revendication 1, caractérisé en ce que l'émetteur de microgouttes est disposé dans un capillaire (1) parcouru par un fluide de dosage variable.

4. Micro-manipulateur pour fluides selon la revendication 1, caractérisé en ce que la diode microfluide (6) se compose d'un microcapillaire ouvert des deux côtés (1) ou d'un système de microcapillaires ouverts des deux côtés (1) disposés serrés les uns à côté des autres, les surfaces des capillaires étant hydrophiles du côté de l'entrée et hydrophobes du côté de la sortie.

5. Micro-manipulateur pour fluides selon la revendication 1, caractérisé en ce que ses composants sont fabriqués en silicium, en verre, en céramique, en métal ou en une combinaison de ces matériaux, et sont fabriqués par un procédé microtechnique et des techniques de construction et de connexion de la technique des microsystèmes.

6. Micro-manipulateur pour fluides selon la revendication 1, caractérisé en ce que la surface de la chambre à goutellettes (5) est hydrophobe.

7. Micro-manipulateur pour fluides selon la revendication 1, caractérisé en ce que plusieurs émetteurs de microgouttes et diodes microfluides (6) sont combinés pour former une cascade à plusieurs couches.

8. Micro-manipulateur pour fluides selon la revendication 1, en particulier pour le dosage contrôlable sans pression de préférence de médicaments hautement concentrés pour l'utilisation en biomédecine, caractérisé en ce qu'entre le réservoir de réserve et l'émetteur de microgouttes se trouve une soupape de sécurité à compensation de pression (8) et en ce que la sortie de la diode microfluide (6) débouche dans une canule de couplage biocompatible (10).
